# EUROPÄISCHE PATENTSCHRIFT

(11) **EP 1 305 055 B1**
(45) Veröffentlichungstag und Bekanntmachung des Hinweises auf die Patenterteilung: **07.04.2004**
(21) Anmeldenummer: 00958344.4
(22) Anmeldetag: 01.08.2000
(51) Int. Cl.: A61L 24/00, A61L 24/04

(54) **WIRKSTOFFHALTIGES IMPLANTAT-BAUSTEIN-SYSTEM UND VERFAHREN ZU DESSEN HERSTELLUNG**
MODULAR IMPLANT SYSTEM CONTAINING ACTIVE SUBSTANCES AND METHOD FOR THE PRODUCTION THEREOF
SYSTEME MODULAIRE D'IMPLANT CONTENANT DES AGENTS ACTIFS ET SON PROCEDE DE FABRICATION

(43) Veröffentlichungstag der Anmeldung: 02.05.2003
(73) Patentinhaber: Coripharm Medizinprodukte GmbH & Co. KG., 64807 Dieburg (DE)
(72) Erfinder: WAHLIG, Helmut, 64287 Darmstadt (DE); DINGELDEIN, Elvira, 63303 Dreieich (DE); BAUER, Jörg, 64293 Darmstadt (DE); WÜST, Edgar, 63110 Rodgau (DE); SATTIG, Christoph, 64807 Dieburg (DE)
(74) Vertreter: Helber, Friedrich G., Dipl.-Ing.
(86) Internationale Anmeldenummer: PCT/EP2000/007426
(87) Internationale Veröffentlichungsnummer: WO 2002/009783

(56) Entgegenhaltungen:
- EP-A- 0 701 824
- WO-A-93/01841
- DE-A- 19 713 229
- DE-A- 19 918 295
- US-A- 4 059 684

## Beschreibung

Die Erfindung betrifft ein Implantat-Baustein-System zur Herstellung eines Implantatmaterials mit einem pulverförmige oder fein granulierte Polyacrylate und/oder Polymethacrylate enthaltenden Grundbaustein und wenigstens einem einen oder mehrere Wirkstoff(e) und/oder Zuschlagstoff(e) enthaltenden Implantat-Baustein, insbesondere für Knochenzemente, unmittelbar vor deren Gebrauch, zur Anwendung in der Orthopädie und Traumatologie.

Der Ersatz erkrankter, schmerzhafter und nicht mehr funktionstüchtiger Gelenke durch künstliche Implantate ist seit vielen Jahren Stand der Technik. Insbesondere der totale Hüftgelenkersatz ist eine der erfolgreichsten und kostengünstigsten Operationen diesem Bereich.

Bezüglich der Verankerungsmöglichkeiten der Gelenkprothesen im Knochenbett stehen grundsätzlich zwei Methoden zur Verfügung. Einmal kann die Fixierung der Prothesenkomponenten durch eine reine Preßpassüng im Knochen ohne irgendwelche weiteren Hilfsstoffe erfolgen. Zum Zweiten werden die Prothesenteile in einem vorher in den Knochenraum eingebrachten Kunststoff eingebettet, wobei der Kunststoff durch Polymerisation im knöchernen Lager aushärtet und so die Prothese dauerhaft fixiert und im Knochen verankert.

Man schätzt die weltweit pro Jahr durchgeführten Hüftgelenkersatz-Operationen auf über 1 Million. Obgleich mehr als 90 % der implantierten Prothesen eine Überlebenszeit von zehn und mehr Jahren besitzen, kommt es aufgrund der sehr großen Gesamtzahl an Eingriffen zu einer nicht unbeträchtlichen Zahl von Komplikationen. Aseptische Lockerungen, tiefe Infektionen und technische Fehler sind die Komplikationen, die am häufigsten zu einer Austauschoperation (Revision) Anlaß geben. Diese Erkenntnis basiert auf Ergebnissen des schwedischen Nationalregisters über revidierte, totale Hüftarthroplastiken, die über einen Untersuchungszeitraum von 1979 bis 1990 mit einem 10-jährigen Follow-up von insgesamt 92.675 primären totalen Hüftendoprothesen-Operationen und einer Rate von 4.858 Erstrevisionen gewonnen wurden (Malchau, H., et al, Institut für Orthopädie der Universität Göteborg, Schweden: "Prognose der totalen Hüftarthroplastik", Annual Meeting der American Academy of Orthopaedic Surgeons 18-23, February 1993, San Francisco, USA). Aufgrund dieser Studie lag die Häufigkeit der verschiedenen Revisionsgründe bei 4.858 Erstrevisionen für aseptische Lockerungen bei 79 %, für tiefe Infektionen bei 9,7 % und für technische Fehler bei 5,9 % (andere Ursachen insgesamt 5,4 %).

Hiernach kommt also auch den Infektionen als dem zweithäufigsten Grund für Revisionen, mit einem Anteil von annähernd 10 %,eine wesentliche Bedeutung zu. Dies ist um so schwerwiegender, als die Behandlung bzw. Sanierung infizierter Endoprothesen den Operateur vor ungleich größere Probleme stellt, die Belastung für den Patienten höher ist, die Behandlungsdauer sehr lange sein kann und die Kosten erheblich höher liegen, als bei den Revisionsoperationen aufgrund aseptischer Lockerungen. Es besteht daher ein dringendes Bedürfnis, den Infektionen prophylaktisch und therapeutisch begegnen zu können.

Nach einer auf Buchholz zurückgehenden Idee ist es bekannt, dem Knochenzement das Antibiotikum Gentamicin zuzusetzen, um sowohl prophylaktisch die Entstehung tiefer Infektionen zu verhüten, als auch bei Revisionsoperationen infizierter Prothesen unter Verwendung eines solchen antibiotikumhaltigen Zements die Sanierung der infizierten Gelenke zu erzielen.

Seit über 20 Jahren ist ein Gentamicin-haltiger Knochenzement auf Basis von Polymethylmethacrylat (Refobacin®-Palacos®R) mit gutem Erfolg in klinischer Anwendung. Im Laufe dieser Zeit haben zahlreiche experimentelle, pharmakokinetische und klinische Untersuchungen die Wirksamkeit dieses Gentamicin-PMMA-Zements bewiesen (u.a. Malchau, H. et al, s.o.). die Anwendung des Gentamicin-haltigen Zements als vorbeugende Maßnahme gegen tiefe Infektionen erwies sich dabei hocheffektiv, mit einer signifikanten Senkung der Infektionsfrequenz.

Aufgrund zahlreicher Untersuchungen erscheint Gentamicin aus bakteriologischer und chemisch-physikalischer Sicht als besonders günstig für die Kombination mit einem PMMA-Knochenzement: Gentamicin besitzt eine vergleichsweise breites antibakterielles Wirkungsspektrum, ist ausreichend hitzestabil und wird aus der Zementmatrix in ausreichenden Mengen freigesetzt.

Trotzdem hat das bekannte Knochenzement-Gentamicin-Gemisch auch wesentliche Nachteile. Zunächst handelt es sich bei diesem Produkt, wie es in der Klinik zur Anwendung kommt, um eine starre Kombination des Polymerpulvers mit nur einem Antibiotikum, eben Gentamicin, in nur einer Dosierung, nämlich 1,25 Gew% bezogen auf das Polymerpulver. Bei der praktischen Anwendung hat sich nun gezeigt, dass diese Dosierung oft nicht ausreicht, um den gewünschten klinischen Effekt zu erzielen. Dies betrifft nicht nur die prophylaktische Anwendung, sondern insbesondere auch die Anwendung des Produkts bei Revisionsoperationen. Hierbei handelt es sich meistens um lang bestehende Krankheitsprozesse, teilweise mit mehreren Vorbehandlungen, und um Infektionen, die auf den Ersteingriff zurückgehen. D.h., die hauptsächliche Infektionsursache sind Keime (oft auch resistente Klinikkeime), die während der Operation, also während des Einsetzens der Endoprothese in die Operationswunde gelangen und sich auf den Metall- und Kunststoffteilen des Prothese ansiedeln. Dabei konnte gezeigt werden, dass die Empfindlichkeit solcher Keime, nachdem sie die Implantate besiedelt haben, gegenüber verschiedenen Antibiotika um ein Vielfaches geringer ist als die der gleichen Keime in der Ursprungspopulation in Suspension.

Ein zweiter Nachteil dieses Gentamicin-haltigen Zements liegt darin, dass einerseits die Gentamicin-Resistenz der ätiologisch verantwortlichen Erreger, zumindest in vielen Kliniken, zugenommen hat. Andererseits hat sich im Laufe der letzten Jahre eine Verschiebung im Erregerspektrum derartiger Infektionserkrankungen hin zu koagulasenegativen Staphylokokken ergeben, die klinisch immer größere Bedeutung erlangen, weil sie in der Mehrzahl multiresistent und in diesem Sinne auch Gentamicin-resistent sind, also nicht mehr im Wirkungsspektrum des Gentamicins liegen. Hinzu kommt, dass auch vermehrt Anaerobier, vor allem anaerobe Kokken, bei Infektionen von Hüftendoprothesen nachgewiesen werden, also Keime, die ebenfalls gegenüber Gentamicin weniger empfindlich bzw. weitgehend resistent sind.

In Anbetracht der Schwere des Krankheitsbildes einer tiefen Infektion, des hohen Gesundheitsrisikos für den Patienten und der besonderen volkswirtschaftlichen Bedeutung dieser Erkrankung, besteht ein dringendes Bedürfnis, sowohl das Antibiotikum bei Bedarf höher dosieren zu können, als auch für die gezielte Bekämpfung der Erreger entsprechend dem ermittelten Antibiogramm und ihrer Antibiotikum-Empfindlichkeit andere Antibiotika als das Gentamicin, die spezifisch wirksamer sind, einsetzen zu können. Das heißt, die Anwendung eines Gentamicin-haltigen Zements, insbesondere in Form einer vorgegebenen, nicht variablen, starren Wirkstoffkonzentration, wird unter den heutigen klinischen Bedingungen den tatsächlichen chemotherapeutischen Erfordernissen nicht mehr gerecht. Der schwerwiegende Nachteil eines handelsüblichen Gentamicin-haltigen Knochenzements liegt darin, dass den sich ständig wandelnden individuellen Problemen in verschiedenen Kliniken und bei einzelnen Patienten mit einem solchen Produkt nicht entsprochen werden kann. Hieraus können sich schwerwiegende therapeutische Nachteile ergeben.

Ein weiterer, schwerwiegender Nachteil der auf dem Markt befindlichen Zemente betrifft die fehlende physikalische Anpassung der Zemente an die individuellen Erfordernisse bei unterschiedlichen Krankheitsbildern verschiedener Patienten.

So steht bei der Entwicklung neuer Zemente oder moderner Applikationssysteme stets nur die Erzielung ausreichend guter mechanischer Kenngrößen für den Zement selbst im Vordergrund. Diese Werte werden zum Beweis einer genügend langen Überlebenszeit der Prothesen herangezogen. Hierbei werden aber weder die verschiedenen Prothesenmaterialien und deren Elastizitätsmodule oder die zur Anwendung kommenden Prothesenformen berücksichtigt, noch werden solche Zemente in ihren mechanischen Eigenschaften den unterschiedlichen Knochenqualitäten und Implantations-Gegebenheiten bei verschiedenen Patienten angepaßt bzw. gerecht.

Bis heute ist kein Knochenzement bekannt, der z.B. an alters- oder krankheitsbedingte Veränderungen der Knochenstruktur (-dichte) besonders angepaßt ist und es dem Operateur ermöglichen würde, individuell und nach Lage des spezifischen Patientenbefundes, unmittelbar vor seiner Anwendung einen entsprechend geeigneten Zement selbst auszuwählen bzw. durch die Komposition der Implantatmasse unter Verwendung hierfür besonders geeigneter Bausteine selbst zusammenzustellen. Ein solcher Zement sollte erfindungsgemäß besondere variable Eigenschaften, z.B. im Hinblick auf die Verarbeitungsmöglichkeiten (geringe, hohe Viskosität), oder die Elastizität (Anpassung der Elastizität des Zements an die Implantatkomponenten), oder die Röntgendichte (Anpassung an die Knochendichte des Patienten), oder die physiologische Organstruktur des Patienten (normaler Knochen, osteoporotischer Knochen, Knochenstruktur beim rheumatischen Formenkreis) besitzen.

Derartige Adaptionen eines Zements, sowohl in bezug auf den Zusatz pharmazeutischer Wirkstoffe als auch im Hinblick auf mechanische Qualitäten sind aber dringend notwendig, weil nur auf diese Weise die derzeit übliche therapeutische Praxis entscheidend verbessert werden kann und es möglich ist, optimale klinisch-therapeutische Behandlungserfolge sicherzustellen.

Die geschilderten Nachteile der heute handelsüblichen Knochenzemente beruhen also auf der Tatsache, dass diese Zemente, die untereinander sowohl in ihrer chemischen Zusammensetzung als auch in ihren physikalischen Eigenschaften sehr ähnlich sind, zwar bei einem Teil der Patienten mit deren pathologischen Gegebenheiten und therapeutischen Erfordernissen konvergieren, bei einem Großteil von Patienten aber deren spezifische therapeutische Anforderungen nicht erfüllen können. So ist es beispielsweise einleuchtend, dass ein bestimmter Zement zwar bei normalen Knochenverhältnissen, wie sie etwa bei Patienten im jüngeren und mittleren Lebensalter überwiegend bestehen, eine bestimmte Prothese mit Erfolg verankern kann, dass dieser Zement aber bei osteolytischen Prozessen, eburnisierten oder osteoporotischen Knochen nicht gleichermaßen wirksam sein kann, weil sich hier die mechanischen Verhältnisse des Knochenlagers wesentlich von denen "normalen" Knochens unterscheiden.

Ebenso klar ist es, dass ein Gentamicin-haltiger Zement zwar bei Knocheninfektionen mit Gentamicin-empfindlichen Keimen mit großer Wahrscheinlichkeit zu einer Sanierung der Infektion führen wird, dass aber in all den Fällen, in denen Gentamicin-resistente Keime für die Infektion verantwortlich sind, dieser Zement versagen muß. Das heißt, Zemente mit vorgegebener physikalischer Potenz und mit einer starren Antibiotikumbeschickung machen einen therapeutisch gezielten Einsatz - je nach individuellen Voraussetzungen - unmöglich. Andererseits würde ein Zement, der in seinen physikalischen Eigenschaften und/oder seiner. Kombination mit pharmazeutischen Wirkstoffen variabel zu gestalten wäre, die vielfältigen therapeutischen Probleme, die such aus dem individuell so unterschiedlichen und vielschichtigen Patientengut ergeben, sicher zu lösen im Stande sein.

So ist es die Aufgabe der nachstehend beschriebenen Erfindung, das Manko eines statischen Systems zu beheben und eine individuelle Patientenversorgung zu ermöglichen. Die Erfindung soll dabei die individuelle Anwendung verschiedener pharmazeutischer Wirkstoffe, insbesondere Antibiotika, in unterschiedlicher Dosierung und/oder Kombination mittels eines gegebenen Implantationsmaterials möglich machen, dabei die Anwendung selbst vereinfachen und verbessern und die Anwendung so gestalten, dass das fertig gemischte Implantationsmaterial den hohen klinischen und pharmazeutischen Anforderungen bezüglich Sterilität, homogener Verteilung der Wirkstoffe, deren standardisierte und reproduzierbarer, protrahierter Freisetzung und dem weitestgehenden Erhalt der Anmischcharakteristik und der mechanischen Eigenschaften des Materials gerecht wird.

Darüber hinaus soll das Implantationsmaterial von Fall zu Fall und je nach klinisch-therapeutischen Erfordernissen, so gestaltet werden können, dass unter Berücksichtigung individueller Patientenparameter beispielsweise der Röntgenkontrast, die Elastizität oder die Viskosität des Implantationsmaterials vom Operateur frei wählbar sind.

Ausgehend von einem Implantat-Baustein-System der eingangs erwähnten Art wird diese Aufgabe erfindungsgemäß dadurch gelöst, dass der den bzw. die Wirkstoff(e) oder Zuschlagstoff(e) enthaltende(n) Implantat-Baustein (e) ebenfalls pulver förmig oder fein granuliert ist bzw. sind und wenigstens einen pulverförmigen oder fein granulierten Wirkstoff oder Zuschlagstoff in höherer Dosierung als der gewünschten Anwendungskonzentration enthält bzw. enthalten, und in der chemischen Zusammensetzung dem Grundbaustein gleicht.

Dem anwendenden Operateur wird damit die Möglichkeit gegeben, entsprechend der gegebenen Operationsbedingungen und nach den individuellen Erfordernissen des zu behandelnden Patienten, selbst und in eigener Entscheidung, unmittelbar vor der Anwendung, variabel zwei oder mehrere verschiedene Implantat-Bausteine in Form eines Baukastensystems zu einem Implantationsmaterial zusammenzustellen.

Das gesamte System von Implantat-Bausteinen umfaßt dabei zweckmäßig die nachstehend noch im einzelnen beschriebenen Implantat-Bausteine mit einem ersten Implantat-Grundbaustein und weiteren, d.h. zweiten, dritten etc. Implantat-Bausteinen, die abhängig vom jeweiligen Anwendungsfall einzeln oder in Kombination mit dem ersten Grundbaustein zum Implantationsmaterial aufbereitet werden.

Der erste Implantat-Grundbaustein (1.) besteht aus einem geeigneten Kunststoff, der vorzugsweise aus Polyacrylat und/oder Polymethacrylat und/oder Kopolymerisaten besteht. Vorzugsweise ist das Implantationsmaterial ein Knochenzement. Die bekannten Knochenzemente werden so zubereitet, dass etwa zwei Teile eines feinteiligen, einen Polymerisationskatalysator (z.B. Dibenzoylperoxid) enthaltenden Präpolymerisates, insbesondere Polymethylacrylat oder eines Mischpolymerisates aus Methylacrylat und Methylmethacrylat, mit einem Teil des flüssigen Monomers, z.B. Acrylsäure oder Methacrylsäuremethylester oder deren Gemische, das einen Beschleuniger (z.B. Dimethyl-p-toluidin) enthält, zu einer formbaren Masse gemischt werden, die in den Körper implantiert wird und dort aushärtet. Solche Knochenzemente sind z.B. unter den Markennamen Palacos®, Sulfix 60®, CMW Bone Cement®, etc. im Handel.

Der erste Implantat-Baustein gemäß einem üblichen Knochenzement steht in verschiedenen Mengen entsprechend den weiteren Bausteinen zur Verfügung, wobei nach dem Mischen eines oder mehrerer verschiedener Implantat-Bausteine mit dem Grundbaustein Pulvermengen entstehen, die vorzugsweise insgesamt etwa 20, 40, 60 oder 80g betragen. Der erste Implantat-Baustein kann dabei bereits eine gewisse Menge an Zuschlagstoffen, wie beispielsweise Röntgenkontrastmittel als Grundstock aufweisen. Ebenso kann er bereits eine Basismenge an pharmazeutischem Wirkstoff, beispielsweise für die Grundprophylaxe, enthalten. Für die Implantation einer Hüftgelenks-Totalendoprothese kann dabei beispielsweise eine Mengenvariation, entsprechend dem Anwendungsziel, für die alleinige Prothesenschaftversorgung oder die Implantation von Schaft und Pfanne, oder, für den Fall einer Revisionsoperation, als Menge des Implantat-Grundbausteins bereitgestellt werden.

Ein zweiter Implantat-Baustein (2.) besteht aus der Matrix des ersten Implantat-Bausteins oder eines beliebigen Implantationsmaterials und einem Röntgenkontrastmittel, welches auf die Patientensituation angepaßt ist. Bei osteoporotischen Knochen (besonders bei älteren weiblichen Patienten) liegt dabei beispielsweise ein schwaches Röntgenkontrastmittel, oder ein Röntgenkontrastmittel in reduzierter Menge, vor. Bei der Behandlung von jungen, vitalen Patienten mit normaler Knochendichte, etwa bei einem (Schenkelhals-)Bruch nach einer Sportverletzung, wird ein starkes Kontrastmittel oder ein solches in hoher Konzentration vorgelegt. Dadurch wird erreicht, dass der Knochenzement in jedem Fall im Röntgenbild sichtbar ist, jedoch durch eine spezifische Anpassung an die gegebene Knochensituation der Heilungsverlauf bei der Röntgenüberwachung nicht überschattet wird. In einer weiteren Variationsmöglichkeit wird eine Spezifikation der Härte des Röntgenkontrastmittels bereitgestellt. So kommt beispielsweise in einem Fall ein weiches, sehr rundes Korn mit niedriger Korngrößenverteilung, welches besonders für die Verwendung weicher Titanprothesen geeignet ist, zur Anwendung, in einem anderen Fall ein härteres Korn mit abgerundeten Ecken, welches eher für die Kobalt-Chrom-Molybdän-Prothesen geeignet ist. Aber auch eine Variation, bei der beispielsweise flüssige Röntgenkontrastmittel, feine Metallpulver, vorzugsweise Tantal-Kugeln oder bioaktive Substanzen, wie beispielsweise Hydroxylapatit, ist denkbar.

Ein dritter Implantat-Baustein (3.) besteht aus der Matrix des ersten Implantat-Bausteins oder eines beliebigen Implantationsmaterials und einem oder mehreren pharmazeutischen Wirkstoffen.

In bevorzugter Ausführungsform der Erfindung ist vorgesehen, dass der oder die Wirkstoffe, bzw. Zuschlagstoffe in höherer, mindestens doppelter Konzentration verglichen mit der gewünschten Anwendungskonzentration enthalten ist/sind, weiter in mindestens 30 % höherer Konzentration.

Bei dem dritten Implantatbaustein werden pharmazeutische Wirkstoffe, vorzugsweise Antibiotika, wie z.B. Gentamicin, Clindamycin, Erythromycin, Vancomycin, Teichoplanin oder allgemein Aminoglycoside, Cephalosporine, Penicilline, Gyrasehemmer, Rifampicin oder andere, Zytostatika, Entzündungshemmer oder ähnliche oder sogenannte Wachstumsfaktoren bzw. -regulatoren in einer deutlich höheren Dosierung als sie der endgültigen klinischen Anwendungskonzentration entspricht, vorzugsweise mit wenigstens einem trockenen pulverförmigen oder fein granulierten Grundstoff, beispielsweise dem des ersten Implantat-Grundbausteins 1. oder eines anderen Implantationsmaterials gemischt. Dies kann das Polymerpulver eines Knochenzements auf Basis von Polyacrylaten und/oder Polymethacrylaten sein.

Ein Vorteil der Erfindung ist, dass durch die Anwendung des erfindungsgemäßen Implantationsbausteins 3. die Wirkstoffmenge, beispielsweise von Antibiotika, im fertigen Implantationsmaterial beliebig und der klinischen Situation entsprechend zu wählen ist, sowie auch individuell das nach den jeweiligen bakteriologischen Voruntersuchungen, beispielsweise dem Antibiogramm, als am geeignetsten ermittelte Antibiotikum ausgewählt oder auch eine Kombination von zweien oder mehreren Antibiotika eingesetzt werden kann. Dabei kommt den Antibiotikumkombinationen, bei der Verwendung von Implantat-Bausteinen, die gemäß dem Implantat-Baustein 3. aufgebaut sind, nach dieser Erfindung große klinische Bedeutung zu. Einerseits ist es möglich, ein Antibiotikum oder mehrere Antibiotika in Form ihrer individuellen Bausteine mit einem Implantationsmaterial-Ausgangsstoff, beispielsweise dem ersten Implantat-Baustein oder einem beliebigen anderen Implantationsmaterial oder einem Knochenzement, in geeigneter Menge zu mischen. Andererseits ist es entsprechend der Erfindung besonders vorteilhaft, zwei oder mehr Antibiotika in einem Implantat-Baustein zusammenzuführen und zu kombinieren. Hierbei besteht darüber hinaus der Vorteil, Antibiotika für eine Kombination auszuwählen, die hinsichtlich ihrer antibakteriellen Aktivitäten eine synergistische Wirkungssteigerung bewirken. Schließlich wird die Gefahr einer Entstehung resistenter Klinikkeime durch die Variation des Antibiotikums gemindert. Eine weitere erfindungsgemäße Möglichkeit besteht darin, Variationen mit schwerlöslichen Substanzen, die eine verzögerte Freisetzung aus der Zementmatrix aufweisen und einen längeren Wirkungszeitraum haben, bis hin zu leicht löslichen Komponenten, mit raschem Wirkungseintritt und hohen, sofort verfügbaren Wirkstoffspiegeln zu verwenden.

Neben den Antibiotika können als Wirkstoffe auch andere pharmazeutische Wirkstoffe oder Zuschlagstoffe eingesetzt werden. Hierdurch ergeben sich verschiedene therapeutische und prophylaktische Anwendungsmöglichkeiten für die Erfindung.

Ein vierter Implantat-Baustein (4.) besteht aus einer Variation von Polymerbestandteilen, durch welche der Elastizitätsmodul des als Knochenzement dienenden Endprodukts, beispielsweise nach Mischen mit dem ersten Implantat-Baustein, beeinflußt werden kann. Dadurch wird es möglich, die Flixibilität des Knochenzements an die Flexibilität des Knochens weitestgehend anzupassen.

Ein fünfter Implantat-Baustein (5.) besteht aus einer Variation von Polymerbestandteilen, die sich in ihrem Anquellverhalten spezifisch unterscheiden und beispielsweise mit dem Implantat-Baustein 1. oder einem anderen Implantationsmaterial gemischt werden können. Durch entsprechende Mischungsverhältnisse ist es dabei möglich, Knochenzemente mit unterschiedlicher Viskosität zu erzielen. So kann z.B. die Palette der verfügbaren Zemente vom niedrig viskösen Zement über eine mittlere Viskosität bis hin zum hochviskösen Zement bereitgestellt werden. Damit ist es möglich, von Fall zu Fall und patientenabhängig die optimale Viskositätsstufe für den anzuwendenden Zement durch den Operateur selbst einzustellen.

Weiterhin kann durch eine derartige Variation die Aushärtezeit des Zements verändert werden. Damit läßt sich gleichzeitig auch die Verarbeitungszeit bzw. -breite des Zements verändern und der jeweiligen Operations- bzw. Patientensituation gezielt anpassen.

Ein sechster Implantat-Baustein (6.) besteht aus dem für die Anmischung der verschiedenen Gemische der Implantat-Bausteine und für die Polymerisation benötigten Monomer, dem gewisse Mengen an Startersubstanzen und Stabilisatoren zugegeben sind. Die benötigten Monomermengen richten sich dabei nach den letztendlich zusammenaddierten Gesamtmengen, bestehend aus den verschiedenen Bausteinen und betragen bei einem üblihen Mischungsverhältnis von 2:1 beispielsweise für Polymerpulvermengen von 20, 40, 60 oder 80g jeweils 10, 20, 30 oder 40 ml.

Es hat sich gezeigt, dass die verschiedenen Einzelbausteine zwar unmittelbar und von Hand miteinander gemischt werden können, dass eine solche Mischart aber insbesondere bezüglich Homogenität und Stabilität erhebliche Nachteile aufweisen kann und deshalb nicht angewandt werden sollte.

Durch eine einfache Mischung der benötigten Komponenten für die Zubereitung der verschiedenen Bausteine von Hand, z.B. unter OP-Bedingungen oder in der Apotheke, können die hohen Qualitätsanforderungen, die an ein Implantationsmaterial gestellt werden müssen, nicht erfüllt werden. Häufig liegen die pharmazeutischen Wirkstoffe oder Zuschlagstoffe auch in Aufbereitungen vor, die sich mit den Implantatmaterial-Ausgangsstoffen der Bausteine nicht hinreichend homogen mischen lassen. Das manuelle Mischen von Implatationsmaterial und Wirk- bzw. Zuschlagstoffen ist daher sogar als gefährlich abzulehnen.

Vorteilhaft ist dagegen, wenn in den Bausteinen 2. bis 5. beispielsweise ein Anteil des Implantat-Bausteins 1. oder eines anderen Implantationsmaterials enthalten ist. Dies bewirkt eine wesentliche Erleichterung der Vermischung, so dass selbst bei Anwendung kurzer Mischzeiten eine homogene Verteilung aller Komponenten untereinander erreicht wird.

Wie die Praxis zeigt, ist es beispielsweise nicht möglich, von Hand - selbst bei Verwendung von Mörser und Pistill - homogene Wirkstoffvermischungen mit dem Polymerpulver von Knochenzementen zu erzielen. Dies gelingt insbesondere dann nicht, wenn die Wirkstoffe in grob kristalliner, stark poröser oder lyophilisierter Form vorliegen, klumpige Konglomerate bilden, wie dies beispielsweise bei hygroskopischen Substanzen der Fall ist, oder harte, scharfkantige Partikel unterschiedlicher Größe enthalten.

Mischungen dieser Art weisen Inhomogenitäten und Fehlstellungen in der ausgehärteten Zementmatrix auf und beeinträchtigen dadurch die mechanische Festigkeit des Implantationsmaterials erheblich. Weiterhin kann bei derartigen Handmischungen die Sterilität nicht in jedem Falle gewährleistet werden.

Bei den Implantat-Bausteinen 2. bis 5. ist es deshalb erfindungsgemäß vorgesehen, dass die pulverförmigen oder fein granulierten Bestandteile vorzugsweise zusammen mit einem bestimmten Anteil der Substanz des Implantat-Bausteins 1. oder eines anderen geeigneten Implantationsmaterials, mit Hilfe eines geeigneten mechanischen Mischgerätes innig vermischt werden, wodurch der oder die Wirkstoff(e) homogen in den Bausteinen verteilt wird bzw. verteilt werden. Als Mischgerät kann vorzugsweise eine Taumelmühle, ein Zwangsmischer oder hierunter bevorzugt ein Wirbelschichtmischer oder Pflugscharmischer verwendet werden. Diese Geräte sind an sich bekannt. Der Mischvorgang kann in an sich bekannter Weise durchgeführt werden und braucht daher hier nicht näher erläutert werden. Es ist jedoch notwendig, die Mischungen vorzugsweise unter Reinraumbedingungen durchzuführen.

Erfindungsgemäß ist es also vorgesehen, dass die Bausteine 2. bis 5. in der Weise zur Herstellung eines Implantationsmaterials verwendet werden, dass sie in wenigstens einem pulverförmigen oder fein granulierten Grundstoff des Implantat-Bausteins 1., oder einem anderen geeigneten Implantationsmaterial, vor dessen weiterer Verarbeitung zu einem gebrauchsfertigen Implantationsmaterial in der für die gewünschte Dosierung der Wirkstoffe erforderlichen Menge eingemischt werden, also beispielsweise in ein wirkstofffreies oder wirkstoffhaltiges Knochenzementpulver auf Polyacrylatoder Polymethylacrylat-Basis.

Dabei hat sich überraschenderweise gezeigt, dass die homogene Vermischung der erfindungsgemäßen Implantat-Bausteine 2. bis 5. beispielsweise mit dem Implantat-Grundstoff des Implantat-Bausteins 1. auf sehr einfache Weise sogar bei üblicher, weniger intensiver Mischung von Hand ohne besondere Hilfsmittel möglich ist, ohne dass die Qualität des Gesamtmaterials durch Konzentrationsgradienten beeinträchtigt würde. Auch die mechanische Mischung in einem Zementmischsystem ist möglich. Schließlich führt auch die Mischung in einem vorgepackten System oder vorzugsweise in einem Vakuum-Anmischsystem ohne weiteres zu einem einwandfreien, völlig homogenen Mischergebnis, wobei vor dem eigentlichen Mischvorgang zu dem vorgelegten Monomer die entsprechende Menge Polymerpulver des Implantat-Bausteins 1. und/oder die Implantat-Bausteine 1., 2., 3., 4. oder 5., oder eine Mischung aus verschiedenen Komponenten der Bausteine 1.-5. zugegeben wird.

Die abschließende Vermischung der Komponenten für das gebrauchsfertige Implantationsmaterial kann in einer abgeschlossenen, besonders dafür eingerichteten Gebrauchseinheit, beispielsweise einem sterilen ein- oder Mehrkammersystem, oder einem entsprechenden Glas- oder Metallbehälter bei Atmosphärendruck oder vermindertem Atmosphärendruck erfolgen. Der Anteil eines oder mehrerer Bausteine der Komponenten 1. bis 5. im Implantat-Baustein 1., der bereits in dem Mischsystem vorgelegt sein kann, beträgt dabei vorzugsweise 5 bis 80 Gew%, weiter vorzugsweise 10 bis 50 Gew% bezogen auf das Gesamtgewicht des Implantationsmaterials.

Unter Verwendung der erfindungsgemäßen Implantat-Bausteine 2 bis 5 eingemischte Knochenzemente werden in analoger Weise so hergestellt, dass eine oder mehrere Komponenten der Bausteine 2. bis 5. mit einem entsprechenden beliebigen Knochenzementpolymerpulver gemischt wird bzw. werden. Für die Fertigstellung des Zements wird ein solches Polymerpulvergemisch im Verhältnis 2:1 mit flüssigem Monomer gemischt.

Die Implantat-Bausteine können nach ihrer Herstellung sterilisiert werden. Es hat sich bei der Prüfung der verschiedenen Einzelbausteine gezeigt, dass nicht jedes Sterilisationsverfahren auf alle Bausteine angewendet werden kann. Dabei ist es jedoch durch den komponentalen Aufbau möglich, unterschiedliche Methoden der Sterilisation anzuwenden, wobei eine Auswahl aus verschiedenen Möglichkeiten, wie beispielsweise Ethylenoxidbegasung, Gammastrahlensterilisation, Betastrahlensterilisation, um nur die gebräuchlichsten zu nennen, erfolgen kann.

Die Herstellung von Knochenzementen unter Verwendung der erfindungsgemäß hergestellten Implantat-Bausteine ermöglicht eine der klinischen Situation angepaßte, im Hinblick auf Wirkstoffauswahl und -dosierung individuelle Beschickung von Knochenzementen und anderen Implantationsmaterialien mit pharmazeutischen Wirkstoffen, vorzugsweise Antibiotika oder Zuschlagstoffen, wobei gleichzeitig das auf diese Weise hergestellte, individuelle Implantationsmaterial die Forderung nach Wahrung der Sterilität und der spezifischen Anmischcharakteristik, homogener Wirkstoffverteilung, optimaler und standardisierter Freisetzung des Wirkstoffs aus der ausgehärteten Matrix und Erhalt der mechanische Eigenschaften erfüllt. Damit steht eine neuartige, sehr wertvolle und vorteilhafte Möglichkeit der Wirkstoffdotierung für die Herstellung wirkstoffhaltiger Implantate, insbesondere Knochenzemente, zur Verfügung.

Im folgenden wird die Erfindung anhand einiger Beispiele noch näher verdeutlicht.

### Beispiel 1 - für Implantat-Baustein 2

10 kg eines beliebigen, feinteiligen Knochenzementpolymerpulvers werden in einem Pflugscharmischer mit 3 kg Zirkondioxid oder Bariumsulfat bis zur homogenen Verteilung des Röntgenkontrastmittels im Zementpulver gemischt. Das Mischen kann auch mit jedem anderen Mischer vorgenommen werden.

### Beispiel 2 - für Implantat-Baustein 2

Es wird analog Beispiel 1 gearbeitet, wobei jedoch 10 kg Polymerpulver und 1 kg Zirkondioxid oder Bariumsulfat eingesetzt werden.

### Beispiel 3 - für Implantat-Baustein 2

Es wird analog Beispiel 1 gearbeitet, wobei jedoch 10 kg Polymerpulver und 1 kg eines feinen Tantalpulvers oder eines anderen Metallpulvers eingesetzt werden.

### Beispiel 4 - für Implantat-Baustein 2

Es wird analog Beispiel 1 gearbeitet, wobei jedoch 10 kg Polymerpulver und 3 kg eines feinen Hydroxylapatitpulvers eingesetzt werden.

### Beispiel 5 - für Implantat-Baustein 2

Es wird analog Beispiel 1 gearbeitet, wobei jedoch 10 kg Polymerpulver und 5 kg eines feinen Hydroxylapatitpulvers eingesetzt werden.

### Beispiel 6 - für Implantat-Baustein 3

Es wird analog Beispiel 1 gearbeitet, wobei jedoch 10 kg Polymerpulver und 1 kg Gentamicin eingesetzt werden.

### Beispiel 7 - für Implantat-Baustein 3

Es wird analog Beispiel 1 gearbeitet, wobei jedoch 10 kg Polymerpulver und 500 g Gentamicin eingesetzt werden.

### Beispiel 8 - für Implantat-Baustein 3

Es wird analog Beispiel 1 gearbeitet, wobei jedoch 10 kg Polymerpulver und 2 kg Gentamicin eingesetzt werden.

### Beispiel 9 - für Implantat-Baustein 3

Es wird analog Beispiel 1 gearbeitet, wobei jedoch 10 kg Polymerpulver und 1 kg Clindamycin eingesetzt werden.

### Beispiel 10 - für Implantat-Baustein 3

Es wird analog Beispiel 1 gearbeitet, wobei jedoch 10 kg Polymerpulver und 500 g Methotrexat eingesetzt werden.

### Beispiel 11 - für Implantat-Baustein 3

Es wird analog Beispiel 1 gearbeitet, wobei jedoch 10 kg Polymerpulver und 2 kg Ampicillin oder Teichoplanin oder Vancomycin oder andere Antibiotika eingesetzt werden.

### Beispiel 13'- für Implantat-Baustein 4

Es wird analog Beispiel 1 gearbeitet, wobei jedoch 10 kg Polymerpulver und 3 kg eines anderen Polymerpulvers, dessen Kopolymer ein hohes Molekulargewicht und einen deutlich höheren Elastizitätsmodul besitzt, eingesetzt werden.

### Beispiel 14 - für Implantat-Baustein 4

Es wird analog Beispiel 1 gearbeitet, wobei jedoch 10 kg Polymerpulver und 5 kg eines anderen Polymerpulvers, dessen Kopolymer ein hohes Molekulargewicht und einen deutlich höheren Elastizitätsmodul besitzt, eingesetzt werden.

### Beispiel 15 - für Implantat-Baustein 5

Es wird analog Beispiel 1 gearbeitet, wobei jedoch 10 kg Polymerpulver mit 5 kg eines anderen Polymerpulvers, dessen Kopolymer ein schnelles Anquellverhalten und eine geringe Viskosität besitzt, eingesetzt werden.

### Beispiel 16 - für Implantat-Baustein 5

Es wird analog Beispiel 1 gearbeitet, wobei jedoch 10 kg Polymerpulver mit 5 kg eines anderen Polymerpulvers, dessen Kopolymer ein langsames Anquellverhalten und eine hohe Viskosität besitzt, eingesetzt werden.

### Beispiel 17 - für die Verwendung eines Implantat-Bausteins in einem Knochenzement

Ein Implantat-Baustein nach einem der Beispiele 1-16 wird durch Begasung oder Bestrahlung sterilisiert und mit einem wirkstofffreien oder wirkstoff- oder zuschlagstoffhaltigen sterilen Knochenzementpolymerpulver, wie es für die Herstellung der Implantat-Bausteine verwendet wird, oder einem beliebigen Knochenzementpulver, im Verhältnis 1+3, 2+2 oder 3+1 Teilen gemischt. Diese Mischung erfolgt in einem dafür geeigneten Anmischsystem, in dem das Knochenzementpolymerpulver vorgelegt ist, vorzugsweise unter verringertem Atmosphärendruck, unmittelbar vor der Anwendung. für die Fertigstellung des Zements wird das Polymerpulvergemisch in einem Verhältnis von etwa 40 g Feststoff zu 20 ml flüssigem Monomer gemischt.

Weitere Beispiele lassen sich für den Fachmann aus der vorangegangenen Beschreibung ableiten.

## Patentansprüche

1. Implantat-Baustein-System zur Herstellung eines Implantatmaterials mit einem pulverförmige oder fein granulierte Polyacrylate und/oder Polymethacrylate enthaltenden Grundbaustein und wenigstens einem einen oder mehrere Wirkstoff(e) und/oder Zuschlagstoff(e) enthaltenden Implantat-Baustein,
**dadurch gekennzeichnet,**
**dass** der den bzw. die Wirkstoff(e) oder Zuschlagstoff(e) enthaltende(n) Implantat-Baustein(e) ebenfalls pulverförming oder fein granuliert ist bzw. sind und wenigstens einen pulverförmigen oder fein granulierten Wirkstoff oder Zuschlagstoff in höherer Dosierung als der gewünschten Anwendungskonzentration enthält bzw. enthalten, und in der chemischen Zusammensetzung dem Grundbaustein gleicht.

2. Wirkstoffhaltiges Implantat-Baustein-System nach Anspruch 1, **dadurch gekennzeichnet, dass** das Implantationsmaterial ein Knochenzement ist, und dass der Grundbaustein für das Implantationsmaterial feinteiliges, einen Polymerisationskatalysator enthaltendes Polyacrylat· und/oder Polymethacrylat ist.

3. Wirkstoffhaltiges Implantat-Baustein-System nach Anspruch 1 oder 2, **dadurch gekennzeichnet, dass** der oder die Wirkstoff(e) in mindestens doppelter Konzentration verglichen mit der gewünschten Anwendungskonzentration im Implantat-Baustein enthalten ist/sind.

4. Wirkstoffhaltiges Implantat-Baustein-System nach einem der Ansprüche 1 oder 2, **dadurch gekennzeichnet, dass** der oder die Wirkstoff(e) in mindestens 30 % höherer Konzentration als der gewünschten Anwendungskonzentration im Implantat-Baustein enthalten ist/sind.

5. Wirkstoffhaltiges Implantat-Baustein-System nach einem der Ansprüche 1 bis 4, **dadurch gekennzeichnet, dass** wenigstens ein Wirkstoff ein Chemotherapeutikum, vorzugsweise ein Antibiotikum, ist.

6. Wirkstoffhaltiges Implantat-Baustein-System nach Anspruch 5, **dadurch gekennzeichnet, dass** das Antibiotikum ausgewählt ist aus der Gruppe Aminoglycosidantibiotika, β-Lactamantibiotika, Clindamycin, Vancomycin, Teicoplanin, Rifampicin.

7. Wirkstoffhaltiges Implantat-Baustein-System nach Anspruch 5, **dadurch gekennzeichnet, dass** als Chemotherapeutikum Methotrexat enthalten ist.

8. Wirkstoffhaltiges Implantat-Baustein-System nach einem der Ansprüche 1 bis 7, **dadurch gekennzeichnet, dass** zwei oder mehr Wirkstoffe enthalten sind.

9. Wirkstoffhaltiges Implantat-Baustein-System nach einem der Ansprüche 1 bis 8, **dadurch gekennzeichnet, dass** der Implantat-Baustein weitere Zuschlag- oder Hilfsstoffe in geringen Mengen enthält.

10. Wirkstoffhaltiges Implantat-Baustein-System nach einem der Ansprüche 1 bis 9, **dadurch gekennzeichnet, dass** die. Korngrößen der Bestandteile des Grundbausteins und des Implantat-Bausteins bzw. der Implantat-Bausteine im Bereich derselben Größenordnung liegen.

11. Implantat-Baustein-System nach Anspruch 9 mit einem wirkstoffhaltigen oder wirkstofffreien Implantat-Baustein, **dadurch gekennzeichnet, dass** der Implantat-Baustein als Zuschlagstoff ein Röntgenkontrastmittel enthält.

12. Implantat-Baustein-System nach Anspruch 11, **dadurch gekennzeichnet, dass** als Röntgenkontrastmittel Bariumsulfat, Zirkondioxid, Hydroxylapatit, Tantal oder andere Metalle in Form eines feinen Pulvers oder Granulats enthalten sind.

13. Implantat-Baustein-System nach Anspruch 9, **dadurch gekennzeichnet, dass** der Zuschlagstoff ein Polyacrylat und/oder Polymethacrylat ist, das einen deutlich höheren oder niedrigeren Elastizitätsmodul besitzt, als das Implantationsmaterial, dem der Implantat-Baustein zugesetzt wird.

14. Implantat-Baustein-System nach Anspruch 9, **dadurch gekennzeichnet, dass** der Zuschlagstoff ein Polyacrylat und/oder Polymethacrylat ist, das sich im Anquellverhalten und der Viskosität deutlich von dem Implantationsmaterial unterscheidet, dem der Implantat-Baustein zugesetzt wird, wobei die Viskosität deutlich höher oder niedriger als die des Implantationsmaterials ist.

15. Verfahren zur Herstellung eines Implantat-Bausteins für das Implantat-Baustein-System nach einem der Ansprüche 1 bis 14, **dadurch gekennzeichnet, dass** die vorgesehenen pulverförmigen oder fein granulierten Bestandteile mit Hilfe eines mechanischen Mischgeräts innig vermischt werden.

16. Verfahren nach Anspruch 15, **dadurch gekennzeichnet, dass** als Mischgerät eine Taumelmühle, ein Zwangsmischer oder vorzugsweise ein Wirbelschichtmischer verwendet wird.

17. Verwendung wenigstens eines Implantat-Bausteins nach einem der Ansprüche 1 bis 14 zur Herstellung eines Implantationsmaterials, **dadurch gekennzeichnet, dass** der oder die Implantat-Baustein(e) in wenigstens einem pulverförmigen oder fein granulierten, den Grundbaustein des Implantationsmaterials bildenden Ausgangsstoff vor dessen weiterer Verarbeitung zu einem gebrauchsfertigen Implantationsmaterial, in der für die gewünschte Dosierung der Wirkstoffoder Zuschlagstoffe erforderlichen Menge eingemischt wird bzw. werden.

18. Verwendung eines oder mehrerer Implantat-Bausteine nach Anspruch 17, **dadurch gekennzeichnet, dass** der oder die Implantat-Baustein(e) vor dem Einmischen in den Ausgangsstoff des Implantationsmaterials sterilisiert wird bzw. werden.

19. Verwendung nach Anspruch 18, **dadurch gekennzeichnet, dass** der oder die Implantat-Baustein(e) vor dem Einmischen in den Ausgangsstoff durch Ethylenoxidbegasung oder Bestrahlung sterilisiert wird bzw. werden.

20. Verwendung eines oder mehrerer Implantat-Bausteine, insbesondere nach einem der Ansprüche 17 bis 19, **dadurch gekennzeichnet, dass** die abschließende Mischung der Komponenten für ein gebrauchsfertiges Implantationsmaterial in einer abgeschlossenen Einheit mittels eines Anmischund/oder Applikationssystems erfolgt.

21. Verwendung eines oder mehrerer Implantat-Bausteine nach Anspruch 19, **dadurch gekennzeichnet, dass** die abschließende Mischung der Komponenten in einem sterilen Plastik- oder Glasbehälter, vorzugsweise unter verringertem Atmosphärendruck erfolgt.

22. Verwendung eines oder mehrerer Implantat-Bausteine nach Anspruch 20 oder 21, **dadurch gekennzeichnet, dass** in der abgeschlossenen Einheit zur Herstellung eines Knochenzements eine pulverförmige oder fein granulierte Feststoffkomponente auf der Basis von Polyacrylat und/oder Polymethacrylat sowie eine flüssige Monomerkomponente vorgelegt wird.

23. Verwendung nach einem der Ansprüche 17 bis 22, **dadurch gekennzeichnet, dass** der Anteil des oder der Implantat-Bausteine 5 bis 80 Gew%, vorzugsweise 10 bis 50 Gew%, bezogen auf das Gesamtgewicht des Implantationsmaterials, beträgt.

## Claims

1. Implantat-component-system for producing an implant material with a basic component containing powdery or finely granular polyacrylates and/or polymethacrylates and at least one implant component containing one or more active agents or additives,
**characterized in**
**that** the implant component(s) containing the powdery or finely granular active agent(s) or additive(s)
is/are also powdery or finely granular and include(s) at least one powdery or finely granular agent or additive in a higher dosage than the desired concentration for the application and has a chemical composition similar or identical to that of the basic component.

2. Implantat-component-system containing according to claim 1, **characterized in that** the implant material is a bone cement and that the basic component for the implant material is a fine-grain polyacrylate and/or polymethacrylate containing a polymerization catalyst.

3. Implant-component-system containing an active agent according to claim 1 or 2, **characterized in that** the active agent(s) is/are contained in the implant component in at least twice the concentration as compared to the concentration desired for the application.

4. Implant-component-system containing an active agent according to claim 1 or 2, **characterized in that** the active agent(s) is/are contained in the implant component in a concentration that is at least 30 % higher than the concentration desired for the application.

5. Implant-component-system containing an active agent according to one of claim 1 to 4, **characterized in that** at least one active agent is a chemotherapeutical agent, preferably an antibiotic.

6. Implant-component-system containing an active agent according to claim 5, **characterized in that** the antibiotic is selected from the group consisting of amino glycoside-antibiotics, β-lactam-antibiotics, clindamycin, vancomycin, teichoplanin, and rifapicin.

7. Implant-component-system containing an active agent according to claim 5, **characterized in that** methotraxate is included as a chemotherapeutical agent.

8. Implant-component-system containing an active agent according to one of claim 1 to 7, **characterized in that** two or more active agents are included.

9. Implant-component-system containing an active agent according to one of claim 1 to 8, **characterized in that** the implant component comprises small quantities of additional additives or accessory agents.

10. Implant-component-system containing an active agent according to one of claim 1 to 9 **characterized in that** the grain sizes of the ingredients of the basic component and of the implant component(s) are of the same order of magnitude.

11. Implant-component-system according to claim 9, **characterized in that** the implant component comprises an x-ray contrast material as an additive.

12. Implant-component-system according to claim 11, **characterized in that** barium sulfate, zirconium dioxide, hydroxyl apatite, tantalum or other metals in form of a fine powder or granulate is/are included as an x-ray contrast material.

13. Implant-component-system according to claim 9, **characterized in that** the additive is a polyacrylate and/or polymethacrylate having a modulus of elasticity that is significantly greater or smaller than that of the implant material to which the implant component is added.

14. Implant-component-system according to claim 9, **characterized in that** the additive is a polyacrylate and/or polymethacrylate having a swelling characteristic and viscosity that are a significantly different from that of the implant material to which the implant component is added, wherein the viscosity is significantly higher or lower than that of the implant material.

15. Method for producing an implant component for the implant-component-system according to one of claims 1 to 14, **characterized in that** the provided powdery or finely granulated components are mixed in a mechanical mixing device.

16. Method according to claim 15, **characterized in that** the mixing device is selected from a mechanical tumbling mill, a positive mixer, or preferably a fluidized bed mixer.

17. Use of an implant component according to one of claims 1 to 14 for producing an implant material **characterized in that** the implant component(s) that contain(s) the active agent(s) or additive(s) is/are mixed in a quantity necessary for the desired dosage of the active agents or additives with at least one powdery or finely granulated material that forms the starting material for the implant material.

18. Use of one or several implant components according to claim 17, **characterized in that** the implant component(s) is/are sterilized before being mixed with the starting material.

19. Use according to claim 18, **characterized in that** the implant component(s) is/are sterilized before being mixed with the starting material by gassing with ethylene oxide or by irradiation.

20. Use of one or several implant components according to one of claims 17 to 19, **characterized in that** the final mixing of the components for a ready-to-use implant material is performed in a sealed unit by using a mixing and/or application system.

21. Use of one or several implant components according to claim 19, **characterized in that** the final mixing of the components is performed in a sterile plastic or glass container, preferably under reduced athmospheric pressure.

22. Use of on or several implant components according to claim 20 or 21, **characterized in that** a powdery or finely granulated solid component based on polyacrylate and/or polymethacrylate as well as a liquid monomer component are provided in the sealed unit.

23. use according to one of claims 17 to 22, **characterized in that** the proportion of the implant component(s) is/are 5 to 80 wt.%, preferably 10 to 50 wt.% referenced to the total weight of the implant material.

## Revendications

1. Système modulaire d'implant en vue de la fabrication d'un matériau d'implantation avec un module de base contenant des polyacrylates et/ou des polyméthacrylates pulvérulents et/ou finement granulés et au moins un module d'implant contenant un ou plusieurs agents actifs et/ou additifs, **caractérisé en ce que** le ou les modules d'implant contenant le ou les agents actifs ou additifs est ou sont également pulvérulents ou finement granulés et contient ou contiennent au moins un agent actif ou additif, pulvérulent ou finement granulé en dosage plus élevé que la concentration d'application souhaitée et est ou sont similaires au module de base pour ce qui est de la composition chimique.

2. Système à modules d'implant, contenant des agents actifs, selon la revendication 1, **caractérisé en ce que** le matériau d'implantation est un ciment pour os et que le module de base pour le matériau d'implantation est un polyacrylate et/ou un polyméthacrylate finement divisé contenant un catalyseur de polymérisation.

3. Système à modules d'implant, contenant des agents actifs, selon la revendication 1 ou 2, **caractérisé en ce que** le ou les agents actifs est ou sont contenus dans une concentration au moins double par comparaison à la concentration d'application souhaitée dans le module d'implant.

4. Système à modules d'implant, contenant des agents actifs, selon la revendication 1 ou 2, **caractérisé en ce que** le ou les agents actifs est ou sont contenus dans une concentration au moins 30% plus élevée que la concentration d'application souhaitée dans le module d'implant.

5. Système à modules d'implant, contenant des agents actifs, selon l'une quelconque des revendications 1 à 4, **caractérisé en ce qu'**au moins un agent actif est un agent chimiothérapeutique, de préférence, un agent antibiotique.

6. Système à modules d'implant, contenant des agents actifs, selon la revendication 5, **caractérisé en ce que** l'agent antibiotique est sélectionné parmi le groupe des antibiotiques aminoglycoside, β-lactame, clindamycine, vancomycine, teicoplanine, rifampicine.

7. Système à modules d'implant, contenant des agents actifs, selon la revendication 5, **caractérisé en ce que** du méthotrexate est contenu en tant qu'agent chimiothérapeutique.

8. Système à modules d'implant, contenant des agents actifs, selon l'une quelconque des revendications 1 à 7, **caractérisé en ce que** deux agents actifs ou plus y sont contenus.

9. Système à modules d'implant, contenant des agents actifs, selon l'une quelconque des revendications 1 à 8, **caractérisé en ce que** le module d'implant contient des additifs ou des adjuvants supplémentaires en quantités faibles.

10. Système à modules d'implant, contenant des agents actifs, selon l'une quelconque des revendications 1 à 9, **caractérisé en ce que** les grosseurs de grain des constituants du module de base et du module d'implant ou des modules d'implant se situent dans le domaine du même ordre de grandeur.

11. Système à modules d'implant selon la revendication 9, avec un module d'implant, contenant des agents actifs ou exempt d'agents actifs, **caractérisé en ce que** le module d'implant contient, en tant qu'additif, un agent de contraste pour rayons X.

12. Système à modules d'implant selon la revendication 11, **caractérisé en ce que** sont contenus, en tant qu'agents de contraste pour rayons X, sous la forme d'une poudre ou d'un granulé fin, le sulfate de baryum, le dioxyde de zirconium, l'hydroxyapatite, le tantale ou d'autres métaux.

13. Système à modules d'implant selon la revendication 9, **caractérisé en ce que** l'additif est un polyacrylate et/ou un polyméthacrylate, qui possède un module d'élasticité nettement plus élevé ou nettement plus bas que le matériau d'implantation, auquel le module d'implant est ajouté.

14. Système à modules d'implant selon la revendication 9, **caractérisé en ce que** l'additif est un polyacrylate et/ou un polyméthacrylate, qui se différencie, en ce qui concerne le comportement de gonflement et la viscosité, nettement du matériau d'implantation, auquel le module d'implant est ajouté, la viscosité étant nettement plus élevée ou nettement plus basse que celle du matériau d'implantation.

15. Procédé en vue de la fabrication d'un module d'implant pour le système de modules d'implant selon l'une quelconque des revendications 1 à 14, **caractérisé en ce que** les constituants prévus pulvérulents ou finement granulés sont mélangés de manière intime à l'aide d'un appareil de mélange mécanique.

16. Procédé selon la revendication 15, **caractérisé en ce que** l'on utilise, en tant qu'appareil de mélange, un malaxeur à excentrique, un malaxeur à mélange forcé ou, de préférence, un malaxeur à couche fluidisée.

17. Utilisation d'au moins un module d'implant selon l'une quelconque des revendications 1 à 14, en vue de la fabrication d'un matériau d'implantation, **caractérisée en ce que** le ou les modules d'implant est ou sont mis en oeuvre dans au moins un matériau de départ pulvérulent ou finement granulé, formant le module de base du matériau d'implantation, avant sa mise en oeuvre ultérieure, pour former un matériau d'implantation prêt à l'emploi, dans lequel l'on ajoute, par mélange, la quantité nécessaire pour le dosage souhaité des agents actifs ou des additifs.

18. Utilisation d'un ou de plusieurs modules d'implant selon la revendication 17, **caractérisée en ce que** le ou les modules d'implant est ou sont stérilisés avant l'addition par mélange à la substance de départ du matériau d'implantation.

19. Utilisation selon la revendication 18, **caractérisée en ce que** le ou les modules d'implant est ou sont stérilisés avant l'addition par mélange à la substance de départ par gazage à l'oxyde d'éthylène ou par irradiation.

20. Utilisation d'un ou de plusieurs modules d'implant, en particulier, selon l'une quelconque des revendications 17 à 19, **caractérisée en ce que** le mélange final des composants pour un matériau d'implantation prêt à l'emploi se fait dans une unité close à l'aide d'un système de mélange et/ou d'application.

21. Utilisation d'un ou de plusieurs modules d'implant selon la revendication 19, **caractérisée en ce que** le mélange final des composants se fait dans un récipient stérile, en matière synthétique ou en verre, de préférence, sous une pression atmosphérique réduite.

22. Utilisation d'un ou de plusieurs modules d'implant selon la revendication 20 ou 21, **caractérisée en ce que**, dans l'unité close en vue de la fabrication d'un ciment pour os, l'on place initialement un composant de substance solide pulvérulent ou finement granulé, à base de polyacrylate et/ou de polyméthacrylate ainsi qu'un composant de monomère fluide.

23. Utilisation selon l'une quelconque des revendications 17 à 22, **caractérisée en ce que** la proportion du ou des modules d'implant est de 5 à 80% en poids, de préférence, de 10 à 50% en poids, par rapport au poids total du matériau d'implantation.
